# EUROPÄISCHE PATENTSCHRIFT

(11) **EP 1 681 564 B1**
(45) Veröffentlichungstag und Bekanntmachung des Hinweises auf die Patenterteilung: **06.05.2009**
(21) Anmeldenummer: 05000619.6
(22) Anmeldetag: 13.01.2005
(51) Int. Cl.: G01N 33/49

(54) **Gerät zum Messen des Gerinnungsverhaltens von Körperflüssigkeiten**
Device for measuring the clotting of body fluids
Dispositif pour déterminer la coagulation de fluides corporels

(43) Veröffentlichungstag der Anmeldung: 19.07.2006
(73) Patentinhaber: Behnk, Holger, D-22417 Hamburg (DE)
(72) Erfinder: Behnk, Holger, D-22417 Hamburg (DE)
(74) Vertreter: Glawe, Delfs, Moll

(56) Entgegenhaltungen:
- DE-A1- 3 135 537
- US-A- 2 034 658
- US-A- 5 203 203
- PATENT ABSTRACTS OF JAPAN Bd. 1998, Nr. 03, 27. Februar 1998 (1998-02-27) & JP 09 304262 A (MATSUSHITA REFRIG CO LTD), 28. November 1997 (1997-11-28)

## Beschreibung

Die Erfindung betrifft ein Gerät zum Messen des Gerinnungsverhaltens von Körperflüssigkeiten, insbesondere Blut, mit einer Küvette, in die die Körperflüssigkeit mit oder ohne Reagenz einfüllbar ist und die ein durch externe Kräfte bewegbares Element aufweist, dessen Änderungen der Bewegungen auf Grund der Gerinnung zur Bestimmung des Gerinnungsverhaltens bestimmt und ausgewertet werden.

Insbesondere bei Operationen ist es erforderlich, das Gerinnungsverhalten von Körperflüssigkeiten, insbesondere von Blut zu bestimmen. Je nach Gerinnungsverhalten müssen unterschiedliche Maßnahmen getroffen werden. In einigen Fällen braucht gar nichts unternommen werden. In anderen Fällen müssen Medikamente verabreicht werden oder andere Maßnahmen getroffen werden.

Ein Verfahren zur Bestimmung des Gerinnungsverhaltens ist dabei die Thrombelastographie (TEG). Bei der Thrombelastographie wird eine frisch abgenommene Blutprobe oder eine Zitratblutprobe mit oder ohne Zusatz von Aktivatoren in das Messsystem eingesetzt. Die Gerinnselfestigkeit wird kontinuierlich bestimmt, indem die Widerstandskraft bestimmt wird, die das Blut zwischen zwei sich relativ zueinander bewegenden Flächen ausübt.

Dieses Verfahren hat gegenüber Laboruntersuchungen den Vorteil, dass es direkt im Operationssaal durchgeführt werden kann. Es ist nicht notwendig, zunächst einen Laufzettel für das Labor auszufüllen, die Probe ins Labor zusenden, dort die Messungen durchzuführen und dann das Ergebnis an den Operateur zu melden. Dieses Verfahren hat auch den Vorteil, dass die Fibrinolyse direkt erfasst werden kann, das heißt, das Auflösen des Gerinnsels, das nach einiger Zeit insbesondere bei Schockzuständen auftreten kann. Bei einem vorbekannten Gerät der Eingangs genannten Art (Prospekt ROTEM Gamma Thromboelastometry, Rapid Evidence Based Therapy of Acute Bleeding) wird das Blut in eine Küvette eingeführt. In die Küvette wird ein Stempel eingeführt, der in Drehbewegungen versetzt werden kann. Die Beeinflussung dieser Drehbewegungen durch das Blutgerinnsel wird aufgezeichnet und ausgewertet.

Die Bedienung eines solchen Gerätes ist sehr kompliziert (Internetausdruck http://www.nobis.at/nobis/bedienu1.htm bis hhttp://www.nobis.at/nobis/bedienu6.htm vom 29. November 2004). Es sind folgende Schritte erforderlich:
1. Der Stempel muss aus einem Vorrat entnommen werden und bis zum Anschlag auf die Achse aufgeschoben werden, die die Drehbewegung bewirken soll.
2. Die Küvette muss in die Küvettenfassung bis zum Anschlag eingedrückt werden.
3. Blut (und gegebenenfalls auch Aktivatoren) müssen in die Küvette pipettiert werden.
4. Bei manueller Pipettierung muss die Zeit gemessen werden. Anschließend muss das Blut kurz mit der Pipettenspitze durchmischt werden.
5. Der Küvettenhalter wird in die Messposition gebracht, wo er in den Stempel geschoben wird.
6. Nach der Messung müssen der Küvettenhalter und der Stempel aus dem Gerät entfernt werden.

Dieses Verfahren und dieses Gerät ist also sehr umständlich und erfordert viele Handgriffe, die nicht oder nur mit Hilfe von aufwendigen Robotern automatisiert werden können.

Ein Gerät gemäß Oberbegriff des Anspruchs 1 wird in DE 3 135 537 beschrieben.

Die Aufgabe der Erfindung besteht in der Schaffung eines Gerätes der eingangs genannten Art, bei dem der Messvorgang auf einfache Weise weitgehend automatisiert werden kann.

Die erfindungsgemäße Lösung besteht darin, dass das bewegbare Element eine magnetisch anziehbare Kugel ist, die auf einer in zur Horizontalen geneigten Ebene in der Küvette bewegbar ist, dass das Gerät einen Magneten zum periodischen Anziehen der Kugel in der geneigten Ebene nach oben und einen Abstandssensor aufweist, mit dem der Ort der Kugel in der Ebene bestimmbar ist.

Die Küvette kann für die Verwendung mit der bereits darin angeordneten Kugel bereitgestellt werden. Nachdem das Blut oder die andere Körperflüssigkeit in die Küvette eingeführt worden ist, kann der Magnet in Betrieb gesetzt werden, der die Kugel zyklisch bewegt. Dadurch kann, falls der Körperflüssigkeit ein Reagenz hinzugesetzt war, die Flüssigkeit durchmischt werden. Jeweils wenn der Magnet abgeschaltet ist, wird die Kugel aufgrund der Schwerkraft auf der schiefen Ebene mit konstanter Kraft schräg nach unten bewegt.

Die Bewegung wird dabei durch den Abstandssensor festgestellt und aufgezeichnet. Setzt die Gerinnung ein, so wird die Kugel nur noch eine sehr kleine Bewegung ausüben. Wird anschließend die Gerinnung aufgelöst (Fibrinolyse) so wird dies durch die wieder größeren Bewegungen der Kugel festgestellt.

Es werden also wesentlich weniger Schritte zur Bestimmung des Gerinnungsverhaltens benötigt, als dies bei den Geräten des Standes der Technik der Fall ist. Eine Automatisierung ist ohne weiteres möglich, da die Küvette nach dem Einfüllen der zu untersuchenden Flüssigkeit nicht erst mit dem beweglichen Element (hier der Kugel) zusammengefügt werden muss, die Kugel kann sich viel mehr von vornherein in der Küvette befinden. Die zyklisch variierende Kraft des Magneten und die Messung durch den Abstandssensor kann automatisch erfolgen.

Bei einer vorteilhaften Ausführungsform wird die Ebene durch zwei gerade parallele Schienen, insbesondere Kanten von Vorsprüngen der Küvette definiert, auf denen die Kugel abrollt. Die Kugel kommt somit nur entlang zwei geraden Linien mit der Küvette in Berührung. Neben der Kugel, unter der Kugel und gegebenenfalls auch über der Kugel kann sich Flüssigkeit befinden.

Damit die Ebene geneigt ist, kann die Ebene in der Küvette geneigt angeordnet sein. Andererseits kann die Ebene in der Küvette senkrecht zu deren Längserstreckung angeordnet sein, wobei das Gerät dann so ausgebildet ist, dass die Küvette geneigt in dieselbe einsetzbar ist, sodass dann ebenfalls die Ebene geneigt ist.

Durch die Neigung der Ebene erhält man wie gesagt eine konstante auf die Kugel wirkende Kraft während der Zeit, während der der Magnet nicht wirkt. Nur diese Kraft und die entsprechende Bewegung der Kugel wird für die Messung berücksichtigt. Die Kraft, mit der der Magnet die Kugel wieder nach oben zieht, wird nicht ausgewertet. Dies hat den Vorteil, das komplizierte Berechnungen von Magnetkräften nicht erforderlich sind.

Vorteilhafterweise weist die Küvette an den Enden der Bewegungsbahn der Kugel eine Berührung der Küvettenwand durch die Kugel verhindernde Vorsprünge auf. Dadurch wird verhindert, dass die Kugel an der Küvettenwand "anklebt".

Zweckmäßigerweise ist der Magnet ein Elektromagnet. Bei einer anderen zweckmäßigen Ausführungsform ist der Magnet ein bewegbarer permanenter Magnet, der zum Beispiel am Umfang einer Scheibe angeordnet ist, die gedreht wird, um so zyklisch eine Magnetkraft auf die Kugel auszuüben.

Als Abstandssensor kann ein induktiver Sensor verwendet werden. Dabei wird ausgenutzt, dass die Kugel magnetisch anziehbar ist, sodass ein induktiver Sensor besonders gut geeignet ist.

Zweckmäßigerweise sind die Küvetten ineinander stapelbar, sodass sie, obwohl sie oben offen sind, bereits mit eingesetzten Kugeln geliefert werden können.

Normalerweise wird das Gerät eine Heizung aufweisen, um die Küvette mit der darin befindlichen Körperflüssigkeit auf einer Temperatur von 37°C zu halten. Bei einer zweckmäßigen Ausführungsform ist aber für spezielle Untersuchungen vorgesehen, dass das Gerät eine Heizeinrichtung mit einstellbarer Temperatur aufweist.

Für die Neigung der Ebene hat sich eine Neigung von ungefähr 20 Grad als besonders vorteilhaft erwiesen. Zweckmäßigerweise sind die Vorsprünge, die die Berührung der Küvettenwand durch die Kugel an den Enden der Bewegungsbahn verhindern, ungefähr einen Millimeter stark. Sie sind zweckmäßigerweise gewölbt, um die Berührungsfläche zwischen Kugel und Vorsprüngen möglichst gering zu machen. Die Schienen, auf denen die Kugel abrollt, sind zweckmäßigerweise in solcher Höhe angeordnet, dass die Kugel auch zum Boden der Küvette einen Abstand von ungefähr einen Millimeter einhält. Die Bewegungen der Kugel sind zweckmäßigerweise auf einen Bereich von ungefähr 2mm begrenzt, um ein Zerreißen des Gerinnsels zu verhindern.

Wenn die Küvette durchsichtig ist und oberhalb der Kugel mit planparallelen Wänden versehen ist, kann die Küvette nicht nur für die Thrombelastographie verwendet werden, sondern auch für optische Messverfahren, zum Beispiel für optische Messungen der Dedimere oder chromogenen Substrate.

Die Erfindung wird im Folgenden anhand einer vorteilhaften Ausführungsform unter Bezugnahme auf die beigefügten Zeichnungen beschrieben. Es zeigen:
- Fig. 1: den prinzipiellen Aufbau eines erfindungsgemäßen Geräts;
- Fig. 2: die Messergebnisse bei der Bestimmung des Gerinnungsverhaltens;
- Fig. 3: die Küvette der Figur 1 von vorne;
- Fig.4: die Küvette der Figur 1 von der Seite;
- Fig.5: die Küvette der Figur 1 von oben.

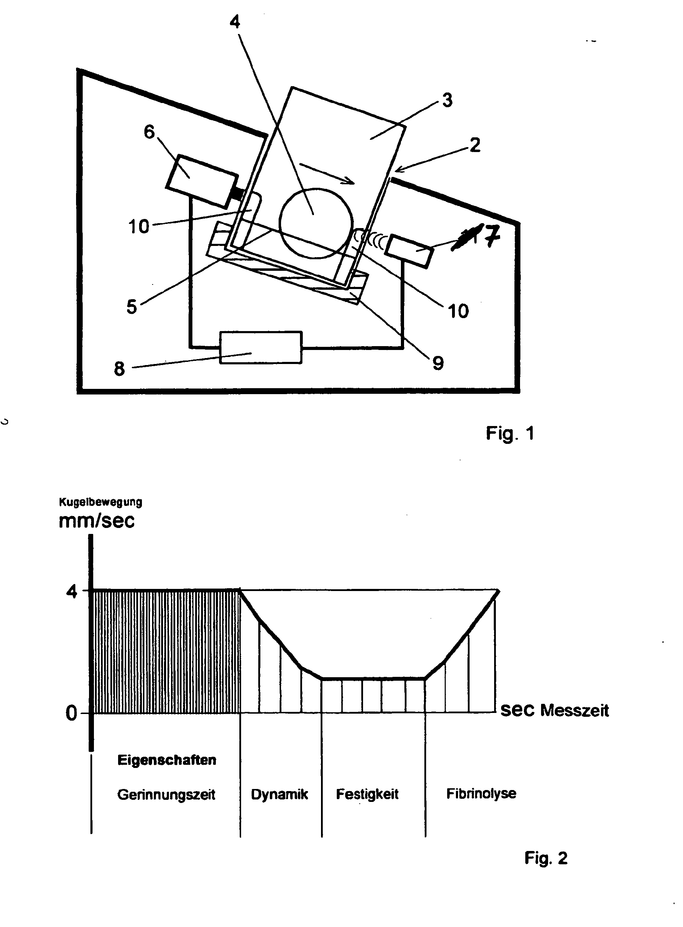

Wie dies in Figur 1 gezeigt ist, weist das Gerät 1 eine Ausnehmung 2 zum Einfügen einer Küvette 3 auf. In der Küvette 3 ist eine Kugel 4 auf einer geneigten Bahn 5 angeordnet. Die Kugel kann dabei durch einen Elektromagneten 6 angezogen werden. Ihr Ort kann durch einen Abstandssensor 7 bestimmt werden. Der Elektromagnet 6 wird durch eine elektronische Schaltung 8 angesteuert, in der auch die Messergebnisse des Sensors 7 ausgewertet werden. Statt eines Elektromagnets 6 könnte auch ein Permanentmagnet vorgesehen sein, der auf geeignete Weise zur Küvette 3 und von der selben weg bewegt wird. Bei 9 sind noch Heizeinrichtungen gezeigt, mit denen die Küvette und ihr Inhalt auf Temperaturen von insbesondere 37°C eingestellt wird. Es ist jedoch auch möglich, Heizeinrichtungen für andere wählbare Temperaturen vorzusehen.

Die Wirkungsweise der in Figur 1 gezeigten Vorrichtung ist die Folgende. Der Elektromagnet wird zunächst mit verhältnismäßig hoher Frequenz von insbesondere mehr als 1 Hz angeregt und zieht jeweils die Kugel 4 an. Nach Abschalten des Elektromagnets rollt die Kugel aufgrund der Schwerkraft wieder nach unten. Durch Vorsprünge 10 wird dabei dafür gesorgt, dass die Kugel 4 nicht an der Wand der Küvette 3 "anklebt". Zu diesem Zweck weist auch die Schiene 5 eine solche Höhe auf, das die Kugel den Boden der Küvette 3 nicht berührt. Die eingefüllte Flüssigkeit wird durch die periodische Bewegung der Kugel umgerührt. Setzt die Gerinnung ein, so wird wegen des erhöhten Widerstandes sich die Kugel verlangsamen und weiterhin nur noch um einen geringeren Betrag bewegen. Das Einsetzen der entsprechenden Änderung definiert die Gerinnungszeit. Da keine großen Vorbereitungshandlungen erforderlich sind und die Flüssigkeit mit Reagenz in die Küvette 3 eingefüllt werden könnte, wenn diese sich bereits im Gerät 1 befindet, kann auch eine sehr kurze Gerinnungszeit von z. B. 10 s gemessen werden.

Nach dem Einsetzen der Gerinnung wird die Frequenz der Kugelbewegung herabgesetzt, damit sich das Gerinnsel aufbauen kann. Während des Aufbaus des Gerinnsels wird dabei die Bewegungsamplitude der Kugel 4 immer geringer. Der Weg der Kugel ist dabei so kurz, dass sich das Fibrinnetz zwischen Kugel und Küvettenwand aufbauen kann, ohne von der Kugel zerstört zu werden. Nach einiger Zeit wird ein stationärer Zustand der Festigkeit erreicht. Unter Umständen tritt aber anschließend Fibrinolyse auf, d. h. dass sich das Gerinnsel wieder auflöst. Die Bewegung der Kugel würde in diesem Falle anwachsen. Das entsprechende Verhalten bzw. der Bewegungsweg der Kugel ist in Fig. 2 dargestellt.

Wie man verstehen wird, bereitet es keine Schwierigkeiten das Gerät zu automatisieren. Es ist auch möglich, innerhalb eines Gerätes Messplätze für mehrere Küvetten vorzusehen. Die Mischung der Probe erfolgt wie erwähnt durch die Kugel, so dass ein anfängliches Mischen nicht erforderlich ist und der Messungsbeginn sehr genau definiert ist. Die Messung ist sehr genau, da aufgrund der Schrägstellung die auf die Kugel 4 wirkende Kraft immer genau definiert und konstant ist. Mit dem erfindungsgemäßen Messgerät kann der Eintritt der Gerinnung, die Dynamik der Gerinnselbildung, die Festigkeit und Stabilität des Gerinnsels wie auch die Fibrinolyse in einem Durchgang gemessen werden.

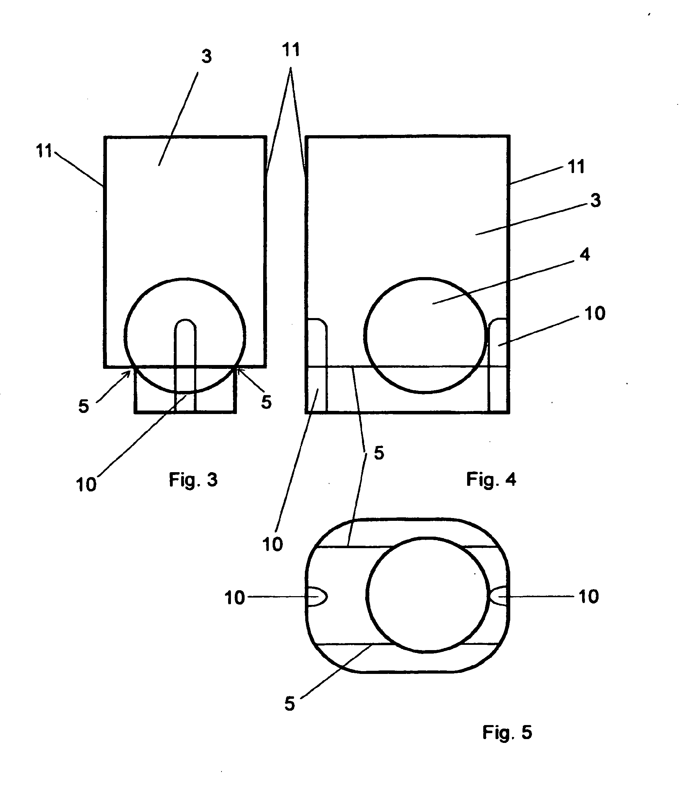

In den Figuren 3 bis 5 sind noch Einzelheiten der Küvette 3 gezeigt. Figur 3 zeigt dabei die Küvette 3 von der Seite, Fig. 4 entsprechend der Figur 1 von vorne und Figur 5 von oben. Wie dies in den Figuren gezeigt ist, sind am Ende der Bewegungsbahn der Kugel 4 halbrunde Vorsprünge 10 vorgesehen, die eine Berührung der Kugel 4 mit der Wand der Küvette 3 und damit ein "Ankleben" verhindern. Aufgrund der Tatsache, dass die Vorsprünge 10 eine halbrunde Oberfläche haben, ist die Berührungsfläche zwischen Kugel 4 und Wand der Küvette 3 sehr klein. Die Bewegungsbahn 5 wird durch eine stufenförmige Verjüngung der Küvette 3 gebildet.

Wie dies in den Figuren 3 und 4 ersichtlich ist, ist die Küvette 3 oberhalb der Kugel 4 mit planparallelen Wänden 11 ausgebildet, so dass hier optische Messungen vorgenommen werden können. Selbstverständlich muss zu diesem Zweck die Küvette 3 durchsichtig sein.

## Patentansprüche

1. Gerät zum Messen des Gerinnungsverhaltens von Körperflüssigkeiten, insbesondere Blut, mit einer Küvette (3), in die die Körperflüssigkeit mit oder ohne Reagenz einfüllbar ist und die ein durch externe Kräfte bewegbares Element (4) aufweist, dessen Änderungen der Bewegungen auf Grund der Gerinnung zur Bestimmung des Gerinnungsverhaltens bestimmt und ausgewertet werden, wobei das bewegbare Element eine magnetisch anziehbare Kugel ist, die auf einer zur Horizontalen geneigten Ebene in der Küvette (3) bewegbar ist und das Gerät einen Magneten (6) zum periodischen Anziehen der Kugel (4) in der geneigten Ebene nach oben aufweist, **gekennzeichnet durch** einen Abstandssensor (7) zur Bestimmung des Ortes der Kugel (4) in der Ebene, und **durch** eine zur Längserstreckung der Küvette (3), welche geneigt in das Gerät (1) eingesetzt ist, senkrechte Anordnung der Ebene.

2. Gerät nach Anspruch 1, **dadurch gekennzeichnet, dass** die Ebene durch zwei gerade parallele Schienen (5), insbesondere Kanten von Vorsprüngen der Küvette (3) definiert ist, auf denen die Kugel (4) abrollt.

3. Gerät nach Anspruch 1 oder 2, **dadurch gekennzeichnet, dass** die Ebene in der Küvette (3) geneigt angeordnet ist.

4. Gerät nach einem der Ansprüche 1 bis 3, **dadurch gekennzeichnet, dass** die Küvette (3) an den Enden der Bewegungsbahn (5) der Kugel eine Berührung der Küvettenwand durch die Kugel (4) verhindernde gewölbte Vorsprünge (10) aufweist.

5. Gerät nach einem der Ansprüche 1 bis 4, **dadurch gekennzeichnet, dass** der Magnet (6) ein Elektromagnet ist.

6. Gerät nach einem der Ansprüche 1 bis 4, **dadurch gekennzeichnet, dass** der Magnet (6) ein beweglicher Permanentmagnet ist.

7. Gerät nach einem der Ansprüche 1 bis 6, **dadurch gekennzeichnet, dass** der Abstandssensor (7) ein induktiver Sensor ist.

8. Gerät nach einem der Ansprüche 1 bis 7, **dadurch gekennzeichnet, dass** die Küvetten (3) ineinander stapelbar sind.

9. Gerät nach einem der Ansprüche 1 bis 8, **dadurch gekennzeichnet, dass** es eine Heizeinrichtung (9) mit einstellbarer Temperatur aufweist.

10. Gerät nach einem der Ansprüche 1 bis 9, **dadurch gekennzeichnet, dass** die Küvetten (3) durchsichtig sind und oberhalb der Kugel mit planparallelen Wänden (11) versehen sind.

## Claims

1. Device for measuring the clotting behaviour of body fluids, in particular blood, having a cuvette (3), into which the body fluid can be filled with or without reagent and which has an element (4) which is movable by external forces and of which the changes of the movements owing to the clotting are determined and evaluated in order to determine the clotting behaviour, the movable element being a magnetically attractable ball which is movable in the cuvette (3) on a plane inclined with respect to the horizontal and the device having a magnet (6) for the periodic attraction of the ball (4) upwards in the inclined plane, **characterised by** a distance sensor (7) for determining the location of the ball (4) in the plane, and by an arrangement of the plane perpendicular to the longitudinal extent of the cuvette (3), which is inserted in an inclined manner into the device (1).

2. Device according to Claim 1, **characterised in that** the plane is defined by two straight parallel rails (5), in particular edges of projections of the cuvette (3), on which the ball (4) rolls.

3. Device according to Claim 1 or 2, **characterised in that** the plane is arranged in an inclined manner in the cuvette (3).

4. Device according to one of Claims 1 to 3, **characterised in that** the cuvette (3) has, at the ends of the movement path (5) of the ball, convex projections (10) which prevent the ball (4) from coming into contact with the cuvette wall.

5. Device according to one of Claims 1 to 4, **characterised in that** the magnet (6) is an electromagnet.

6. Device according to one of Claims 1 to 4, **characterised in that** the magnet (6) is a movable permanent magnet.

7. Device according to one of Claims 1 to 6, **characterised in that** the distance sensor (7) is an inductive sensor.

8. Device according to one of Claims 1 to 7, **characterised in that** the cuvettes (3) can be stacked in one another.

9. Device according to one of Claims 1 to 8, **characterised in that** it has a heating device (9) with adjustable temperature.

10. Device according to one of Claims 1 to 9, **characterised in that** the cuvettes (3) are transparent and are provided with plane-parallel walls (11) above the ball.

## Revendications

1. Appareil de mesure du comportement à la coagulation de fluides corporels, notamment du sang, avec une cuvette (3) dans laquelle le fluide corporel peut être mis avec ou sans réactif et qui présente un élément (4) mobile sous l'effet d'une force extérieure, dont les variations de mouvements en raison de la coagulation sont déterminées et évaluées pour la détermination du comportement à la coagulation, l'élément mobile étant une boule pouvant être magnétiquement attirée, qui peut bouger dans la cuvette (3) sur un plan incliné par rapport à l'horizontale, l'appareil comportant un aimant (6) pour attirer la boule (4) périodiquement vers le haut dans le plan incliné, **caractérisé par** un détecteur de distance (7) pour déterminer l'emplacement de la boule (4) dans le plan et par une disposition, perpendiculaire par rapport à l'extension longitudinale de la cuvette, du plan, qui est disposé inclinés dans l'appareil (1).

2. Appareil selon la revendication 1, **caractérisé en ce que** le plan est défini par deux rails droits parallèles (5), notamment des bords en saillie de la cuvette (3) sur lesquels roule la boule.

3. Appareil selon la revendication 1 et 2, **caractérisé en ce que** le plan est disposé incliné dans la cuvette (3).

4. Appareil selon l'une des revendications 1 à 3, **caractérisé en ce que** la cuvette (3) présente aux extrémités de la piste de déplacement (5) de la boule des saillies (10) destinées à empêcher tout contact de la paroi par la boule (4).

5. Appareil selon l'une des revendications 1 à 4, **caractérisé en ce que** l'aimant (6) est un électro aimant.

6. Appareil selon l'une des revendications 1 à 5, **caractérisé en ce que** l'aimant (6) est un aimant permanent mobile.

7. Appareil selon l'une des revendications 1 à 6, **caractérisé en ce que** le détecteur de distance (7) est un détecteur inductif.

8. Appareil selon l'une des revendications 1 à 7, **caractérisé en ce que** les cuvettes (3) sont empilables les unes dans les autres.

9. Appareil selon l'une des revendications 1 à 8, **caractérisé en ce qu'**il présente un dispositif de chauffage (9) avec réglage de température.

10. Appareil selon l'une des revendications 1 à 9, **caractérisé en ce que** les cuvettes (3) sont transparentes et pourvues au-dessus de la boule de parois (11) planes et parallèles.
